# EUROPÄISCHE PATENTSCHRIFT

(11) **EP 0 728 725 B1**
(45) Veröffentlichungstag und Bekanntmachung des Hinweises auf die Patenterteilung: **09.02.2000**
(21) Anmeldenummer: 96101795.1
(22) Anmeldetag: 08.02.1996
(51) Int. Cl.: C07C 45/62, C07C 49/233

(54) **Verfahren zur Herstellung von 1-(4-Chlorphenyl)-4,4-dimethyl-pentan-3-on**
Process for the preparation of 1-(4-chlorophenyl)-4,4 dimethyl-pentane-3-one
Procédé pour la préparation de 1-(4-chlorophényl)-4,4-diméthyl-pentane-3-one

(30) Priorität: 21.02.1995 DE 19505938
(43) Veröffentlichungstag der Anmeldung: 28.08.1996
(73) Patentinhaber: BAYER AG, 51368 Leverkusen (DE)
(72) Erfinder: Darsow, Gerhard, Dr., D-47804 Krefeld (DE)

(56) Entgegenhaltungen:
- DE-A- 3 702 301
- DE-C- 4 004 031
- FR-A- 1 478 704

## Beschreibung

Die vorliegende Erfindung betrifft ein neues kostengünstiges, kontinuierlich arbeitendes Verfahren zur Herstellung von 1-(4-Chlorphenyl)-4,4-dimethyl-pentan-3-on, bei welchem nur sehr geringe Mengen der üblicherweise bei der Hydrierung von 1-(4-Chlorphenyl)-4,4-dimethyl-pent-1-en-3-on als Nebenprodukte entstehenden Stoffe 1-(4-Chlorphenyl)-4,4-dimethyl-pentan-3-ol und 1-Phenyl-4,4-dimethylpentan-3-on gebildet werden.

1-(4-Chlorphenyl)-4,4-dimethyl-pentan-3-on ist ein wichtiges Ausgangsprodukt für die Herstellung von 1-(4-Chlorphenyl)-3-(1,2,4-triazol-1-yl-methyl)-4,4-dimethylpentan-3-ol, das ausgezeichnete fungizide und pflanzenwuchsregulierende Eigenschaften besitzt (EP-A-0 040 345).

Es ist bekannt, 1-(4-Chlorphenyl)-4,4-dimethyl-pentan-3-on diskontinuierlich durch Hydrierung von 1-(4-Chlorphenyl)-4,4-dimethyl-pent-1-en-3-on in organischen Lösungsmitteln, wie Methanol, Toluol, Xylol, Cyclohexan, Isooctan, Ethern oder Estern, über Ni-haltigen Katalysatoren in Pulverform herzustellen (EP-A 0 354 991).

Es ist ferner bekannt 1-(4-Chlorphenyl)-4,4-dimethyl-pentan-3-on diskontinuierlich durch Hydrierung von 1-(4-Chlorphenyl)-4,4-dimethyl-pent-1-en-3-on über Ni in Pulverform herzustellen, wobei in Gegenwart von Toluol oder Xylol als Lösungsmittel (DE-OS 4 004 031) gearbeitet wird; das Ausgangsmaterial wird beispielsweise durch Kondensation von 4-Chlor-benzaldehyd mit 3,3-Dimethyl-butan-2-on hergestellt.

Schließlich ist aus FR-A 1 478 704 ein Verfahren zur Hydrierung von ungesättigten Carbonylverbindungen bekannt, bei dem trägerfreie oder geträgerte Katalysatoren eingesetzt werden, die Metalle der VIII. und der V1. Nebengruppe des Periodensystems in stückiger Form enthalten. Die erzielbaren Selektivitäten sind dabei akzeptabel, die erzielbaren Aktivitäten nicht.

Die vorliegende Erfindung betrifft ein ökologisch und technisch vorteilhaftes Verfahren zur Herstellung von 1-(4-Chlorphenyl)-4,4-dimethyl-pentan-3-on aus 1-(4-Chlorphenyl)-4,4-dimethyl-pent-1-en-3-on, das dadurch gekennzeichnet ist, daß man 1-(4-Chlorphenyl)-4,4-dimethyl-pent-1-en-3-on in Lösung von Mono-Alkoholen kontinuierlich mit einer 10- bis 40-fachen Menge von Wasserstoff unter einem Druck von 50 bis 400 bar und bei einer Temperatur von 30 bis 160°C über als Hydrierungskatalysatoren dienenden, in einem Reaktor als Festbett angeordneten trägerfreien Formkörpern aus verpreßten Metallpulvern von (i) einem oder mehreren Elementen der Eisenuntergruppe der VIII. Nebengruppe des Periodensystems hydriert, wobei die Metallpulver gegebenenfalls zusätzlich mit (ii) aktivierend wirkenden Elementen der VI. Nebengruppe legiert oder gemischt sind und (iii) gegebenenfalls weiterhin eines oder mehrere hydrier-inerte Elemente aus der Gruppe von Aluminium, Silizium, Kohlenstoff und Titan enthalten, wobei die Formkörper eine Druckfestigkeit von 20 bis 250 N auf ihre Oberfläche und eine innere Oberfläche von 10 bis 80 m²/g aufweisen.

Der Reaktionsverlauf läßt sich durch das folgende Reaktionsschema veranschaulichen:

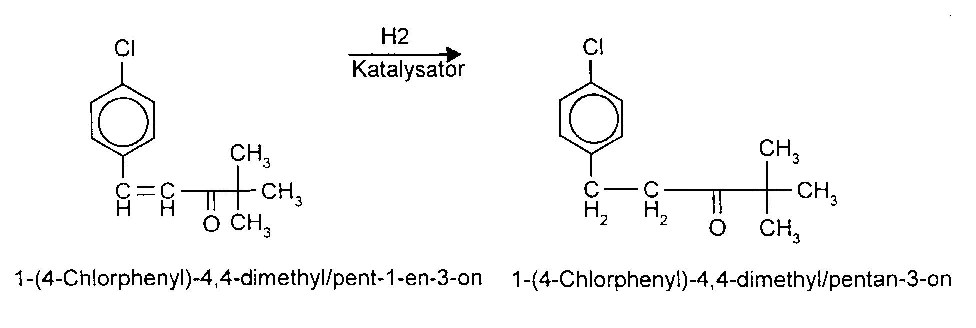

Bei den bekannten Verfahren zur Herstellung von 1-(4-Chlorphenyl)-4,4-dimethylpentan-3-on werden Pulver-Suspensionsverfahren angewendet, bei denen das 1-(4-Chlorphenyl)-4,4-dimethyl-pent-1-en-3-on in einem Lösungsmittel über pulverförmigen Katalysatoren mit Wasserstoff hydriert wird.

Diskontinuierliche Verfahren haben den Nachteil, daß ihre Kapazität relativ zum Reaktionsvolumen sehr klein ist und somit ein Bedarf nach großen Reaktionsapparaturen und Lagertanks besteht. Energieverbrauch und Personalbedarf sind verhältnismäßig hoch.

Kontinuierliche Pulverkatalysatorverfahren, die mit einem oder mehreren in Kaskase geschalteten Hydrierreaktoren arbeiten, vermeiden einen Teil dieser Nachteile. Es bleibt jedoch das Erfordernis, die pulverförmigen Katalysatoren gezielt zu dosieren, umzupumpen und quantitativ vom Reaktionsprodukt abzufiltrieren. Die Katalysatorschlammpumpen unterliegen einem hohen mechanischen Verschleiß. Die quantitative Entfernung der pulverförmigen Katalysatoren aus dem Reaktionsprodukt ist aufwendig. Ferner ist die Gefahr groß, die Katalysatoraktivität durch die zusätzlichen Operationen verhältnismäßig schnell zu verringern. Es ist daher wünschenswert, weil vorteilhaft, die Reaktion über fest angeordneten Katalysatoren ablaufen zu lassen. Solche Katalysatoren müssen eine hohe Aktivität besitzen, die über einen längeren Zeitraum nicht nachlassen darf, weil häufige Katalysatorwechsel bei Festbettreaktionen ebenfalls aufwendig sind.

Zum Einsatz kommt 1-(4-Chlorphenyl)-4,4-dimethyl-pent-1-en-3-on mit einer Reinheit >99 %. Es lassen sich aber auch Destillationsrückläufe mit geringeren Konzentrationen von 1-(4-Chlorphenyl)-4,4-dimethyl-pent-1-en-3-on einsetzen.

Monoalkohole für das erfindungsgemäße Verfahren sind vor allem solche mit 1 bis 4 C-Atomen, wie Methanol, Ethanol, Propanol, i-Propanol, Butanol, i-, sec-, und tert.-Butanol, bevorzugt Methanol und Ethanol, besonders bevorzugt Methanol.

Akohole mit mehr als 4-C-Atomen und Diole sind als Reaktionsmedium grundsätzlich geeignet, sind aber teurer und wegen ihrer Siedepunktslage schwieriger vom Reaktionsprodukt abzutrennen. Gemische der genannten Alkohole können ebenfalls eingesetzt werden. 1-(4-Chlorphenyl)-4,4-dimethyl-pent-1-en-3-on kann als 15 bis 20 Gew.-%ige Lösung in den genannten Alkoholen eingesetzt werden.

Hierbei kann es vorteilhaft sein, die Lösung mit geringen Alkalimengen (NaOH, KOH, Na₂CO₃, K₂CO₃ u.ä. in einer Menge von 0,1 bis 0,2 g/l) auf einen pH-Wert von 8 bis 10 einzustellen und ihr eine kleine Menge (0,1 bis 0,2 g/l) einer organischen Schwefelverbindung, z.B. Bis-(2-hydroxy-ethyl)-sulfid, zuzusetzen.

Die Druckfestigkeit der trägerfreien Formkörper kann nach DIN 50 106 bestimmt werden. Die Überprüfung von trägerfreien Formkörpern auf die anspruchgemäßen inneren Oberflächen und damit auf Brauchbarkeit für das erfindungsgemäße Verfahren können nach Methoden durchgeführt werden, die von F.M. Nelsen und F.T. Eggertsen, Analyt. Chem. 30 (1958), S. 1387-1390 bzw. S.J. Gregg und S.W. Sing, Adsorption, Surface Area and Porosity, London 1982, Kap. 2 und 6, beschrieben worden sind.

Die Eisenuntergruppe der VIII. Nebengruppe des Periodensystems enthält die Metalle Eisen, Kobalt und Nickel. Die erfindungsgemäß zu verwendenden trägerfreien Formkörper enthalten (i) eines oder mehrere dieser Metalle in Mengen von mindestens 60, vorzugsweise 70, insbesondere mindestens 80 Gew.-%, bezogen auf die trägerfreien Formkörper. Fe, Co und Ni liegen hierbei untereinander in beliebigen Verhältnissen vor. In bevorzugter Weise enthalten die Formkörper Ni als (i) und zwar in einer Menge von 60 bis 100 Gew.-%, besonders bevorzugt 80 bis 100 Gew.-% aller Metalle (i).

Die VI. Nebengruppe des Periodensystems enthält die Elemente Chrom, Molybdän und Wolfram. Die erfindungsgemäß zu verwendenden trägerfreien Formkörper können (ii) eines oder mehrere dieser Metalle enthalten. Diese Metalle (ii) liegen in einer Menge von Null bis zu 15 Gew.-% vor, bezogen auf die trägerfreien Formkörper. In bevorzugter Weise enthalten die Formkörper eines oder mehrere dieser Metalle in Mengen von 0,1 bis 15 Gew.-%, besonders bevorzugt 0,3 bis 10 Gew.-%, ganz besonders bevorzugt 0,5 bis 5 Gew.-%, bezogen auf die trägerfreien Formkörper.

Die erfindungsgemäß zu verwendenden trägerfreien Formkörper können darüber hinaus, jeweils bezogen auf trägerfreie Formkörper (iii) Null bis 25 Gew.-%, vorzugsweise Null bis 15 Gew.%, besonders bevorzugt Null bis 10 Gew.-% anderer hydrier-inerter Elemente aus der Gruppe von Aluminium, Silicium, Kohlenstoff und Titan enthalten. Nach einer besonders bevorzugten Ausführungsform enthalten die trägerfreien Formkörper außer den Komponenten (i) und (ii) nicht mehr als 8 Gew.-% Aluminium und nicht mehr als 5 Gew.-% anderer Elemente.

Die Herstellung der trägerfreien Formkörper kann nach gebräuchlichen Methoden durch Verpressen der Metallpulver auf Tablettier- oder Pelletiermaschinen unter hohem Druck erfolgen, wobei zur Verbesserung des Haftvermögens der Metallpartikel auch Graphit in Mengen von 0,5 bis 1,5 Gew.-%, bezogen auf das Gesamtgewicht der den Katalysator bildenden Bestandteile, oder Klebstoffe in kleinen Mengen zum Einsatz kommen können. Die Herstellung der trägerfreien Formkörper wird vorzugsweise in einer sauerstofffreien Atmosphäre erfolgen, um Oberflächenoxidationen zu vermeiden. Am wirksamsten und für die Reaktionsführung am günstigsten sind tablettierte oder pelletierte Formkörper mit Abmessungen von 2 bis 7 mm, bevorzugt 3 bis 5 mm. Von erheblicher Bedeutung ist die Druckfestigkeit der Formkörper, die erfindungsgemäß bei Werten von 20 bis 250 N, bevorzugt 110 bis 220 N, liegt. Niedrigere Druckfestigkeiten führen zu Formkörperzerfall bzw. erosivem Abrieb, was eine metallische Kontaminierung des Reaktionsproduktes bewirken würde. Von erheblicher Bedeutung ist weiterhin die innere Oberfläche der Formkörper, die erfindungsgemäß bei Werten von 10 bis 80 m²/g liegt und ausschlaggebend für einen möglichst quantitativen Umsatz der Einsatzstoffe ist. Die Formkörper haben makroskopisch eine glatte Oberfläche.

Für den Hydrierprozeß wird auf einen Druck von 50 bis 400 bar, bevorzugt 100 bis 300 bar, vorkomprimierter reiner Wasserstoff eingesetzt, wobei man mit 10 bis 40-fachen molaren Wasserstoffüberschüssen arbeitet.

Die Hydrierung erfolgt kontinuierlich im Festbettverfahren an den als Hydrierungskatalysatoren dienenden trägerfreien Formkörpern der beschriebenen Art, indem man das zu hydrierende, in Alkohol gelöste 1-(4-Chlorphenyl)-4,4-dimethyl-pent-1-en-3-on entweder im Gleichstrom mit dem zuvor zugemischten Wasserstoff von unten nach oben aufsteigend über die in den Hydrierreaktor gefüllten Formkörper strömen läßt oder dem von oben einströmenden Wasserstoff von unten kommend entgegenführt (Gegenstromverfahren).

Der Hydrierprozeß wird bei Temperaturen von 30 bis 160°C, vorzugsweise 40 bis 80°C, durchgeführt. Niedrigere Temperaturen bedingen höhere Verweilzeiten oder den Verzicht auf einen quantitativen Umsatz des 1-(4-Chlorphenyl)-4,4-dimethylpent-1-en-3-on. Höhere Temperaturen führen zu vermehrter Bildung von Nebenprodukten. Die Ausgangsmaterialien werden vorzugsweise vor Eintritt in den Reaktor auf 30 bis 70°C, besonders bevorzugt 40 bis 60°C vorgewärmt.

Die stündliche Katalysatorbelastung kann 100 bis 300 g 1-(4-Chlorphenyl)-4,4-dimethyl-pent-1-en-3-ol/l Katalysator betragen.

Der Hydrierreaktor kann entweder ein einzelnes Hochdruckrohr aus Stahl oder einer Stahllegierung sein, das mit dem trägerfreien Formkörpern ganz oder teilweise gefüllt wird, wobei auch die Anwendung auf Horden (Drahtkörbe o.ä.) nützlich sein kann, oder aber ein ummanteltes Hochdruckrohrbündel, dessen Einzelrohre mit Formkörpern ganz oder teilweise gefüllt werden.

Unter den geschilderten Reaktionsbedingungen sind ganz unerwartet hohe Katalysatorstandzeiten von 15 000 Stunden und mehr zu erzielen, was zu bisher bei der Hydrierung von 1-(4-Chlorphenyl)-4,4-dimethyl-pent-1-en-3-on noch nicht erreichten Katalysatorverbräuchen <0,1 Gew.-% führt.

Das den Hydrierreaktor verlassende Reaktionsgemisch besteht nach der Entspannung, bei welcher man den überschüssigen Wasserstoff abfangen und nach erfolgter Komprimierung und Ergänzung von verbrauchtem Wasserstoff erneut zum Einsatz bringen kann, und der destillativen Entfernung des Lösungsmittels bei einem 99,9 bis 100 %igen Umsatz des Einsatzstoffes zu mehr als 99 Gew.-% aus 1-(4-Chlorphenyl)-4,4-dimethyl-pentan-3-on. Es kann organische Verunreinigungen in einer Menge bis zu maximal 0,8 Gew.-% enthalten.

Das erzeugte 1-(4-Chlorphenyl)-4,4-dimethyl-pentan-3-on kann nach der destillativen Entfernung der Verunreinigungen in einer Reinheit von ≥ 99,9 Gew.-% erhalten werden und ist in dieser Reinheit für alle weiterverarbeitenden Prozesse einsetzbar.

### Beispiele

### Beispiel 1

Ein senkrecht stehendes, wärmeisoliertes Hochdruckrohr aus nichtrostendem Stahl von 45 mm Innendurchmesser und 1 m Länge wurde mit 1,4 1 eines durch Tablettierung von Nickelpulver hergestellten Hydrierungskatalysators gefüllt, der bei einer Zylinderhöhe von 3 mm und einem Durchmesser von 3 mm eine Druckfestigkeit von 147 N auf die Zylindermantelfläche und eine innere Oberfläche von 63 m²/g aufwies. Durch dieses Rohr wurden gemeinsam mit der 20-fachen molaren Menge von unter einem Druck von 300 bar stehendem, hochreinem Wasserstoff stündlich 1 400 ml einer 18 Gew.-%igen Lösung von 1-(4-Chlorphenyl)-4,4-dimethyl-pent-1-en-3-on mit einem Reinheitsgrad >99 Gew.-% in reinem Methanol, die zusätzlich 0,2 Gew.-% NaOH und 0,2 Gew.-% Bis-(2-hydroxy-ethyl)-sulfid enthielt, kontinuierlich gepumpt und zwar von unten nach oben aufsteigend.

Zu hydrierende Lösung und Wasserstoff wurden vorab gemeinsam durch einen Wärmeaustauscher geführt und so hoch erhitzt, daß sie in das Hochdruckrohr mit einer Temperatur von 55°C eintraten. Das das Hochdruckrohr verlassende Gemisch aus flüssigem Reaktionsprodukt und überschüssigem Wasserstoff wurde in einen Abscheider geführt, von wo der Wasserstoff nach Einsatz der verbrauchten Menge wieder zusammen mit neuer zu hydrierender Lösung in den Verwärmer und von da erneut in das Hochdruckrohr gepumpt wurde.

Nach erfolgter Hydrierung und destillativer Entfernung des Lösungsmittels wurde das Reaktionsprodukt gaschromatographisch untersucht. Es enthielt 0,35 Gew.-% 1-(4-Chlorphenyl)-4,4-dimethyl-pentan-3-ol und 0,04 Gew.-% 1-Phenyl-4,4-dimethyl-pentan-3-on. Der Gehalt an 1-(4-Chlorphenyl)-4,4-dimethyl-pentan-3-on lag bei 99,21 Gew.-% (Rest zu 100 % = Ausgangsmaterial und unbekannte Nebenprodukte).

Nach der destillativen Entfernung der Verunreinigungen wurde das gewonnene 1-(4-Chlorphenyl)-4,4-dimethyl-pentan-3-on in einer Reinheit von 99,9 Gew.-% erhalten.

Der Katalysator war nach einer Laufzeit von 5 400 Stunden unverändert wirksam, so daß sich die Zusammensetzung des Reaktionsproduktes über diesen Zeitraum nicht veränderte.

### Beispiel 2

In einem Hochdruckrohr wie in Beipiel 1 wurde bei einer Temperatur von 60°C und einem Wasserstoffdruck von 200 bar der Wasserstoff im umgekehrten Reaktionsfluß wie in Beispiel 1 der aufsteigenden zu hydrierenden Lösung entgegengeführt, wobei stündlich eine gleich große Menge wie in Beispiel 1 hydriert wurde. Der Katalysator war durch Tablettierung einer pulverisierten Nickel-Eisen-Legierung gewonnen wurden. Die Legierung enthielt einen Eisenanteil in Nickel von 15 Gew-%. Die Tabletten hatten bei einer Zylinderhöhe von 5 mm und einem Durchmesser von 5 mm eine Druckfestigkeit von 137 N auf die Zylindermantelfläche und eine innere Oberfläche von 74 m²/g.

Nach einer Laufzeit von 2 400 Stunden lag der Umsatz des eingesetzten 1-(4-Chlorphenyl)-4,4-dimethyl-pent-1-en-3-ons bei 99,95 Gew.-%. Der Gehalt an 1-(4-Chlorphenyl)-4,4-dimethyl-pentan-3-ol lag bei 0,3 Gew.-% und der Gehalt an 1-Phenyl-4,4-dimethyl-pentan-3-on bei 0,06 Gew.-%. Der Gehalt an 1-(4-Chlorphenyl)-4,4-dimethyl-pentan-3-an betrug 99,24 Gew.-% (Rest zu 100 % Ausgangsmaterial und unbekannte Nebenprodukte).

Nach der destillativen Entfernung der Verunreinigungen wurde das gewonnene 1-(4-Chlorphenyl)-4,4-dimethyl-pentan-3-on in einer Reinheit von 99,95 Gew.-% erhalten.

### Beispiel 3

Ein senkrecht stehendes, wärmeisoliertes Hochdruckrohr aus nichtrostendem Stahl von 45 mm Innendurchmesser und 1 m Länge wurde mit 1,4 l eines durch Tablettierung von Pulver einer Ni/Mo-Legierung mit einem Mo-Gehalt von 1,75 % hergestellten Hydrierungskatalysators gefüllt, der bei einer Zylinderhöhe von 5 mm und einem Durchmesser von 5 mm eine Druckfestigkeit von 191 N und eine innere Oberfläche von 58 m²/g aufwies. Durch dieses Rohr wurden gemeinsam mit der dreißigfachen molaren Menge von unter einem Druck von 300 bar stehendem hochreinem Wasserstoff stündlich 1 600 ml einer 1-(4-Chlorphenyl)-4,4-dimethylpent-1-en-3-on Lösung wie in Beispiel 1 gepumpt und zwar von unten nach oben aufsteigend.

Methanolische Lösung und Wasserstoff wurden vor Eintritt in das Hochdruckrohr auf eine Temperatur von 40°C gebracht.

Nach einer Laufzeit von 2 800 Stunden lag der Umsatz des eingesetzten 1-(4-Chlorphenyl)-4,4-dimethyl-pent-1-en-3-ons bei 100 Gew.-%. Der Gehalt an 1-(4-Chlorphenyl)-4,4-dimethyl-pentan-3-ol lag bei 0,1 Gew.-% und der Gehalt an 1-Phenyl-4,4-dimethyl-pentan-3-on bei 0,05 Gew.-%. Der Gehalt an 1-(4-Chlorphenyl)-4,4-dimethyl-pentan-3-an betrug 99,45 Gew.-% (Rest zu 100 % unbekannte Nebenprodukte).

### Beispiel 4

In einem Hochdruckrohr wie in Beispiel 1, aber aus Hochdruckstahl N 9, wurde bei einer Temperatur von 45°C und einem Wasserstoffdruck von 300 bar stündlich eine gleich große Menge einer 17 Gew.-%igen Lösung von 1-(4-Chlorphenyl)-4,4-dimethyl-pent-1-en-3-on in Methanol hydriert. Der Katalysator wurde durch Tablettierung von Pulver einer Ni/Mo-Legierung mit einer Mo-Gehalt von 1,02 Gew.-% und einem Al-Gehalt von 5,1 Gew.-% hergestellt. Die Tabletten hatten bei einer Zylinderhöhe von 5 mm und einem Durchmesser von 5 mm eine Druckfestigkeit von 210 N und eine innere Oberfläche von 71 m²/g.

Nach einer Laufzeit von 4 200 Stunden lag der Umsatz des eingesetzten 1-(4-Chlorphenyl)-4,4-dimethyl-pent-1-en-3-ons bei 100 Gew.-%. Der Gehalt an 1-(4-Chlorphenyl)-4,4-dimethyl-pentan-3-on im lösungsmittelfreien Reaktionseluat betrug 99,64 Gew-%. Der Gehalt an 1-(4-Chlorphenyl)-4,4-dimethyl-pentan-3-ol lag bei 0,15 Gew.-% und der Gehalt an 1-Phenyl-4,4-dimethyl-pentan-3-on bei 0,04 Gew.-% (Rest zu 100 % unbekannte Nebenprodukte).

### Beispiel 5

In einem Hochdruckrohr wie in Beispiel 1 wurde bei einer Temperatur von 60°C und einem Wasserstoffdruck von 300 bar stündlich eine gleich große Menge einer 17 Gew.-%igen Lösung von 1-(4-Chlorphenyl)-4,4-dimethyl-pent-1-en-3-on in Methanol hydriert, die zusätzlich 0,1 Gew.-% NaOH und 0,2 Gew.-% Bis-(2-hydroxy-ethyl)-sulfid enthielt. Der Katalysator wurde durch Tablettierung von Ni-Pulver, das zusätzlich 5,8 Gew.-% Al enthielt, gewonnen. Die Tabletten hatten bei einer Zylinderhöhe von 3 mm und einem Durchmesser von 3 mm eine Druckfestigkeit von 168 N und eine innere Oberfläche von 68 m²/g.

Nach einer Laufzeit von 5 300 Stunden lag der Umsatz des eingesetzten 1-(4-Chlorphenyl)-4,4-dimethyl-pent-1-en-3-ons bei 100 Gew.-%. Der Gehalt an 1-(4-Chlorphenyl)-4,4-dimethyl-pentan-3-on im lösungsmittelfreien Reaktionseluat lag bei 99,38 Gew.-%. Der Gehalt an 1-(4-Chlorphenyl)-4,4-dimethyl-pentan-3-ol lag bei 0,2 Gew.-% und der Gehalt an 1-Phenyl-4,4-dimethyl-pentan-3-on bei 0,02 Gew.-% (Rest zu 100 % unbekannte Nebenprodukte).

Nach der destillativen Entfernung der Verunreinigungen wurde das gewonnene 1-(4-Chlorphenyl)-4,4-dimethyl-pentan-3-on in einer Reinheit von 99,9 Gew.-% erhalten.

## Patentansprüche

1. Verfahren zur Herstellung von 1-(4-Chlorphenyl)-4,4-dimethyl-pentan-3-on aus 1-(4-Chlorphenyl)-4,4-dimethyl-pent-1-en-3-on, dadurch gekennzeichnet, daß man 1-(4-Chlorphenyl)-4,4-dimethyl-pent-1-en-3-on in Lösung von Mono-Alkoholen kontinuierlich mit einer 10- bis 40-fachen Menge von Wasserstoff unter einem Druck von 50 bis 400 bar und bei einer Temperatur von 30 bis 160°C über als Hydrierungskatalysatoren dienenden, in einem Reaktor als Festbett angeordneten trägerfreien Formkörpern aus verpreßten Metallpulvern von (i) einem oder mehreren Elementen der Eisenuntergruppe der VIII. Nebengruppe des Periodensystems hydriert, wobei die Metallpulver gegebenenfalls zusätzlich mit (ii) aktivierend wirkenden Elementen der VI. Nebengruppe legiert oder gemischt sind und (iii) gegebenenfalls weiterhin eines oder mehrere hydrier-inerte Elemente aus der Gruppe von Aluminium, Silizium, Kohlenstoff und Titan enthalten, wobei die Formkörper eine Druckfestigkeit von 20 bis 250 N auf ihre Oberfläche und eine innere Oberfläche von 10 bis 80 m²/g aufweisen.

2. Verfahren nach Anspruch 1, dadurch gekennzeichnet, daß die Formkörper eine Druckfestigkeit von 110 bis 220 N aufweisen.

3. Verfahren nach Anspruch 1, dadurch gekennzeichnet, daß die Formkörper, makroskopisch betrachtet, eine glatte Oberfläche aufweisen.

4. Verfahren nach Anspruch 1, dadurch gekennzeichnet, daß die Formkörper zylindrisch- oder kugelförmig sind und Durchmesser von 2 bis 7 mm besitzen.

5. Verfahren nach Anspruch 1, dadurch gekennzeichnet, daß die Hydriertemperatur im Festbettreaktor 40 bis 80°C liegt.

6. Verfahren nach Anspruch 1, dadurch gekennzeichnet, daß eine Lösung von 1-(4-Chlorphenyl)-4,4-dimethyl-pent-1-en-3-on in Methanol den Hydrierreaktor von unten nach oben aufsteigend passiert.

7. Verfahren nach Anspruch 1, dadurch gekennzeichnet, daß die Formkörper mindestens 60 Gew.-%, bezogen auf die trägerfreien Formkörper, an Metallen (i) enthalten.

8. Verfahren nach Anspruch 1, dadurch gekennzeichnet, daß die Formkörper als (i) Ni in einer Menge von 60 bis 100 Gew.-%, bevorzugt 80 bis 100 Gew.-%, bezogen auf die trägerfreien Formkörper, enthalten.

9. Verfahren nach Anspruch 1, dadurch gekennzeichnet, daß die Formkörper Null bis 15 Gew.-% Metalle (ii), bezogen auf die trägerfreien Formkörper, enthalten.

10. Verfahren nach Anspruch 1, dadurch gekennzeichnet, daß die Formkörper Null bis 25 Gew.-% Elemente (iii), bezogen auf die trägerfreien Formkörper, enthalten.

## Claims

1. Process for preparing 1-(4-chlorophenyl)-4,4-dimethyl-pentan-3-one from 1-(4-chlorophenyl)-4,4-dimethyl-pent-1-en-3-one, characterized in that 1-(4-chlorophenyl)-4,4-dimethyl-pent-1-en-3-one in solution of monoalcohols is hydrogenated continuously with a 10-fold to 40-fold amount of hydrogen under a pressure of from 50 to 400 bar and at a temperature of from 30 to 160°C over support-free shaped bodies consisting of compressed metal powders of (i) one or more elements of the iron subgroup of transition group VIII of the Periodic Table, the metal powders optionally additionally being alloyed or mixed with (ii) elements of transition group VI which have an activating action and optionally further contain (iii) one or more hydrogenation-inert elements from the group of aluminium, silicon, carbon and titanium, with the shaped bodies being arranged in the fixed bed in a reactor and serving as hydrogenation catalysts and having a compressive strength of from 20 to 250 N on their surface and an internal surface area of from 10 to 80 m²/g.

2. Process according to Claim 1, characterized in that the shaped bodies have a compressive strength of from 110 to 220 N.

3. Process according to Claim 1, characterized in that the shaped bodies, viewed macroscopically, have a smooth surface.

4. Process according to Claim 1, characterized in that the shaped bodies are cylindrical or spherical and have a diameter of from 2 to 7 mm.

5. Process according to Claim 1, characterized in that the hydrogenation temperature in the fixed-bed reactor is from 40 to 80°C.

6. Process according to Claim 1, characterized in that a solution of 1-(4-chlorophenyl)-4,4-dimethyl-pent-1-en-3-one in methanol passes through the hydrogenation reactor from the bottom upwards.

7. Process according to Claim 1, characterized in that the shaped bodies contain at least 60% by weight, based on the support-free shaped bodies, of metals (i).

8. Process according to Claim 1, characterized in that the shaped bodies contain as (i) Ni in an amount of from 60 to 100% by weight, preferably from 80 to 100% by weight, based on the support-free shaped bodies.

9. Process according to Claim 1, characterized in that the shaped bodies contain from zero to 15% by weight of metals (ii), based on the support-free shaped bodies.

10. Process according to Claim 1, characterized in that the shaped bodies contain from zero to 25% by weight of elements (iii), based on the support-free shaped bodies.

## Revendications

1. Procédé de préparation de 1-(4-chlorophényl)-4,4-diméthyl-pentan-3-one à partir de 1-(4-chlorophényl)-4,4-diméthyl-pent-1-èn-3-one, caractérisé en ce qu'on hydrogène la 1-(4-chlorophényl)-4,4-diméthylpent-1-èn-3-one en solution d'alcools monofonctionnels, en continu, avec des quantités de 10 à 40 fois d'hydrogène sous une pression de 50 à 400 bars et une température de 30 à 160°C en utilisant comme catalyseurs d'hydrogénation, placés en lit compact dans un réacteur, des corps moulés sans support de poudres métalliques comprimées de (i) un ou plusieurs éléments du sous-groupe du fer du groupe voisin VIII du système périodique des éléments, les poudres métalliques étant éventuellement en outre sous forme d'alliage ou mélangées avec (ii) des éléments agissant comme activateurs du groupe voisin VII et contenant (iii) éventuellement en outre un ou plusieurs éléments inertes à l'hydrogénation dans le groupe de l'aluminium, le silicium, le carbone et le titane, les corps moulés présentant une résistance à la pression de 20 à 250 N sur leur surface et une surface interne de 10 à 80 m²/g.

2. Procédé selon la revendication 1, caractérisé en ce que les corps moulés présentent une résistance à la pression de 110 à 220 N.

3. Procédé selon la revendication 1, caractérisé en ce que les corps moulés présentent une surface lisse considérée macroscopiquement.

4. Procédé selon la revendication 1, caractérisé en ce que les corps moulés ont une forme cylindrique ou sphérique et présentent un diamètre de 2 à 7 mm.

5. Procédé selon la revendication 1, caractérisé en ce que la température d'hydrogénation dans le réacteur à lit solide est de 40 à 80°C.

6. Procédé selon la revendication 1, caractérisé en ce qu'une solution de 1-(4-chlorophényl)-4,4-diméthyl-pent-1èn-3-one dans le métahnol passe de manière ascendante du bas vers le haut dans le réacteur d'hydrogénation.

7. Procédé selon la revendication 1, caractérisé en ce que les corps moulés contiennent au moins 60% en poids, rapporté aux corps moulés sans support, de métaux (i).

8. Procédé selon la revendication 1, caractérisé en ce que les corps moulés contiennent comme (i) du Ni en quantité de 60 à 100% en poids, de préférence 80 à 100% en poids, rapporté aux corps moulés sans support.

9. Procédé selon la revendication 1, caractérisé en ce que les corps moulés contiennent de 0 à 15% en poids de métaux (i), rapporté aux corps moulés sans support.

10. Procédé selon la revendication 1, caractérisé en ce que les corps moulés contiennent de 0 à 25% en poids d'éléments (iii), rapporté aux corps moulés sans support.
